# EUROPEAN PATENT SPECIFICATION

(11) **EP 2 412 714 B1**
(45) Date of publication and mention of the grant of the patent: **11.11.2015**
(21) Application number: 10755518.7
(22) Date of filing: 18.03.2010
(51) Int. Cl.: C07D 471/16, A61K 31/519, A61P 21/02, A61P 23/02

(54) **METHOD FOR THE INDUSTRIAL PURIFICATION OF BIOLOGICALLY ACTIVE PHYCOTOXINS**
VERFAHREN ZUR INDUSTRIELLEN REINIGUNG BIOLOGISCH AKTIVER PHYCOTOXINE
PROCÉDÉ DE PURIFICATION INDUSTRIELLE DE PHYCOTOXINES BIOLOGIQUEMENT ACTIVES

(30) Priority: 24.03.2009 CL 7232009
(43) Date of publication of application: 01.02.2012
(73) Proprietor: Proteus S.A., Santiago (CL)
(72) Inventor: LAGOS GONZÁLEZ, Marcelo Santiago, Santiago (CL)
(74) Representative: Carvajal y Urquijo, Isabel
(86) International application number: PCT/IB2010/051187
(87) International publication number: WO 2010/109386

(56) References cited:
- WO-A1-2005/110275
- WO-A2-98/43619
- US-A1- 2003 100 574
- US-A1- 2008 006 579
- LUBNA ZAMAN ET AL: "Occurrence of paralytic shellfish poison in Bangladeshi freshwater puffers", TOXICON, vol. 35, no. 3, 1 March 1997 (1997-03-01), pages 423-431, XP55036381, ISSN: 0041-0101, DOI: 10.1016/S0041-0101(96)00167-5
- EDWARD J. SCHANTZ ET AL: "The Purification and Characterization of the Poison Produced by Gonyaulax catenella in Axenic Culture", BIOCHEMISTRY, vol. 5, no. 4, 1 April 1966 (1966-04-01), pages 1191-1195, XP55036404, ISSN: 0006-2960, DOI: 10.1021/bi00868a011
- GHAZAROSSIAN V E ET AL: "Identification of a poison in toxic scallops from a Gonyaulax tamarensis red tide", BIOCHEMICAL AND BIOPHYSICAL RESEARCH COMMUNICATIONS, ACADEMIC PRESS INC. ORLANDO, FL, US, vol. 59, no. 4, 19 August 1974 (1974-08-19), pages 1219-1225, XP024777990, ISSN: 0006-291X, DOI: 10.1016/0006-291X(74)90444-6 [retrieved on 1974-08-19]
- Il Shik Shin ET AL: "Reestablishment of Approval Toxin Amount in Paralytic Shellfish Poison-Infested Shellfish", J. Fd Hyg. Safety, 31 December 1998 (1998-12-31), pages 143-148, XP55036412, Retrieved from the Internet: URL:http://210.101.116.28/W_ftp41/14600812 _pv.pdf [retrieved on 2012-08-27]
- CASTRO, D. ET AL.: 'The effect of temperature on growth and production of paralytic shellfish poisoning toxins by the cyanobacterium Cylindrospermopsis raciborskii C10.' TOXICON. vol. 44, no. 5, October 2004, ISSN 0041-0101 pages 483 - 489
- ROSITANO, J. ET AL.: 'Ozonation of NOM and algal toxins in four treated waters.' WATER RESEARCH. vol. 35, no. 1, January 2001, ISSN 0043-1354 pages 23 - 32
- GUEVREMONT, R. ET AL.: 'Comparison of cation-exchange and chelating cation-exchange resins for the concentration of saxitoxin.' ANALYTICA CHIMICA ACTA. vol. 255, no. 1, December 1991, ISSN 0003-2670 pages 163 - 168
- SAYFRITZ, S. J. ET AL.: 'Determination of paralytic shellfish poisoning toxins in Norwegian shellfish by liquid chromatography with fluorescence and tandem mass spectrometry detection.' TOXICON. vol. 52, no. 2, August 2008, ISSN 0041-0101 pages 330 - 340
- W.W. Carmichael: "Cyanobacteria secondary metabolites-the cyanotoxins", Journal of Applied Bacteriology, vol. 72, no. 6, 1 June 1992 (1992-06-01), pages 445-459, XP055086109, ISSN: 0021-8847, DOI: 10.1111/j.1365-2672.1992.tb01858.x
- CANTOR C R ET AL: "MAPPING THE STRUCTURE OF MACROMOLECULAR ASSEMBLIES BY COMBINING CHEMICAL MODIFICATION AND SEPARATION METHODS", JOURNAL OF INDUSTRIAL MICROBIOLOGY AND BIOTECHNOLOGY, BASINGSTOKE, GB, vol. 17, no. 5-6, 1 January 1996 (1996-01-01), pages 373-384, XP009093880, ISSN: 1367-5435, DOI: 10.1007/BF01574768
- W.W. CARMICHAEL: "Cyanobacteria secondary metabolites-the cyanotoxins", JOURNAL OF APPLIED BACTERIOLOGY, vol. 72, no. 6, 1 June 1992 (1992-06-01), pages 445-459, XP055086109, ISSN: 0021-8847, DOI: 10.1111/j.1365-2672.1992.tb01858.x

## Description

### FIELD OF THE INVENTION

This invention is related to the industrial production of the paralyzing phycotoxins neosaxitoxin, saxitoxin and gonyaulatoxins (gonyaulatoxin 2 and gonyaulatoxin 3) in large amounts, under controlled conditions and continuously from cyanobacteria that produce paralyzing phycotoxins, and particularly to the purification of said phycotoxins. The central objective of this is having a substantially pure compound that retains its potent biological activity until the end product, wherein it is used as a raw material for the development of new **medicines.**

These phycotoxins can be applied in cosmetic or pharmaceutical products, *e.g.* in cosmetic products to fight wrinkles and expression lines or in pharmaceutical products for clinical application such as local anesthetics, medicines to control pathologies associated to muscular hyperactivity, to control pain both at the local and peripheral level, *i.e.* products to improve life quality.

### DESCRIPTION OF THE PREVIOUS ART

The phycotoxins neosaxitoxin, saxitoxin and gonyaulatoxins are active compounds produced by harmful algae blooms of the genera *Alexandrium* sp., *Piridinium* sp., and *Gimnodinium* sp., (Lagos, N. (1998) Microalgal blooms: A global issue with negative impact in Chile. Biol. Res. 31: 375-386). In the last 15 years, it has been demonstrated that these phycotoxins can also be produced by fresh water cyanobacteria such as photosynthetic blue-green algae, besides being produced by marine dinoflagellates.

Until now, only 4 genera of cyanobacteria able to produce paralyzing phycotoxins have been identified, and each produces a different mixture of phycotoxins both in amounts and in types of phycotoxins produced, *i.e.* they produce different profiles of paralyzing phycotoxins (Lagos, N., Onodera, H., Zagatto, P.A., Andrinolo, D., Azevedo, S.M.F.Q., and Oshima, Y., 1999, The first evidence of paralytic shellfish toxins in the freshwater cyanobacterium Cylindrospermopsis raciborskii, isolated from Brazil. TOXICON, 37 : 1359 - 1373. Pereira, P., Onodera, H., Andrinolo, D., Franca, S., Araujo, F., Lagos, N., and Oshima, Y., 2000, Paralytic shellfish toxins in the freshwater cyanobacteria Aphanizomenon flos-aquae, isolated from Montargil reservoir, Portugal. TOXICON, 38: 1689 - 1702).

The active principle of these paralyzing phycotoxins acts as a specific blocker of the voltage-dependent sodium channels present in excitable cells (Kao, C.Y., 1966, Tetrodotoxin, saxitoxin and their significance in the study of excitation phenomenon. Pharm. Rev. 18: 997-1049). Due to the inhibition of sodium channels, the transmission of a nervous impulse is blocked and in this way the release of neurotransmitters is prevented at the level of the neuromotor junction, which prevents muscular contraction. Due to these physiological effects, these compounds are potentially useful in pharmacology when used as muscular activity inhibitors in pathologies associated to muscular hyperactivity, such as muscular spasms and focal dystonias, when applied locally in injectable form. Additionally, since a blockage of the nervous impulse at the transmission level is generated when these compounds are applied as a local infiltration, they are not only able to block the efferent neurotransmission pathways, but also block afferent pathways and in this way they cause an inhibition of the sensory pathways and generate an anesthetic effect, being both effects inseparable when these compounds are locally injected. This is a surprising effect, since both effects are simultaneous (US Patent 4,001,413).

The phycotoxins neosaxitoxin, saxitoxin and gonyaulatoxins are not commercially available in this moment as a massive product in this moment, despite their big potential applications with therapeutic and cosmetic ends. It is evident that an industrial production procedure for these compounds is needed to satisfy their growing demand related to the large amount of uses and clinical and cosmetic applications recently developed.

Lubna Zaman describes the isolation of several phycotoxins including saxitoxin and gonyautoxin, by retaining the compounds on charcoal with following elution and HPLC (Lubna Zaman, Osamu Arakawa, Ako Shimosu and Yoshio Onoue, 1997, Occurrence of paralytic shellfish poison in Bangladeshi freshwater puffers. TOXICON, 35, 3, 423-431). However, the process includes extraction in ethanol and HCl medium al pH 2. Phycotoxins lose their activity at low pH, resulting the HCl medium additionally difficult to remove from the purified product.

US 2008/006579 A1 refers to a general method to concentrate biotransformed products catalyzed by cells in diatomic earth, wherein the desired products are found in the medium outwards said cells. Again, at the described pH<4 values phycotoxins are not active, whereas higher pH values are reported to yield insufficient at the publication's method. The present invention, on the contrary, lysates the cell in order to obtain the intracellular phycotoxins, and does so maintaining the biological activity of the said phycotoxins.

Ghazzarossian describes a method for the isolation of saxitoxin employing celite but also acidifying with HCl to pH 2-3 (Ghazzarossian V.E., Schantz E.J., Schnoes H.K. and Strong F.M., 1974, Identification of a poison in toxic scallops from a Gonyaulax Tamarensis red tide. Biochm. and Biophys. Research Comunic. Academic Press Inc., 59, 4, 1219-1225).

Remarkable is that both Lubna Zaman as Ghazzarossian purify phycotoxins from infected fish tissue as source, which determines the extraction process.

The invention presented herein is directed to the extraction, fractioning and purification of the paralyzing phycotoxins neosaxitoxin, saxitoxin and gonyaulatoxins, starting from an innovative process to continuously culture cells under controlled conditions and in large amounts from cyanobacterial strains (blue-green algae). The central objective is to keep the potent biological activity of this active principle during all the industrial purification process.

The cyanobacteria able to produce paralyzing phycotoxins belong to the genera: *Cylindrospermopsis sp, Microcystis sp, Anabaena sp, Gomphosphaeria sp, Oscillatoria sp, Aphanizomenon sp* (*Aphanizomenon issatchenkoi, Aphanizomenon flos-aquae, Aphanizomenon gracile*), *Lyngbya wollei* and others. None of these cyanobacteria has been used until now to produce phycotoxins at industrial levels.

The industrial production of phycotoxins from cyanobacteria must solve two technical problems that are interrelated but different from each other. First, a large amount of phycotoxin-producing biomass must be generated. This technical problem is solved by the invention protected in the Patent Application CL 722-2009, also presented by the authors of the present invention. Second, once the biomass and phycotoxins are produced, these compounds must be purified under conditions suitable to keep the pharmacological activity of the active compounds, preserve their potent biological activity and deliver massive yields. This second technical problem is solved by the present invention.

The proposed method is the purification of phycotoxins from a clonal culture of cyanobacteria that produces a simple profile of paralyzing phycotoxins (comprising one or two phycotoxins or having a profile with one phycotoxin that represents more than 75% of the total profile composition), *e.g*. a strain that produces only neosaxitoxin and saxitoxin or only gonyaulatoxins 2/3 as major components. The achievement of yields higher than 75% of the active pharmacological principle is also a surprising effect, which is attained in the present industrial procedure.

The chemical structure of these phycotoxins has a general structure (I) and its particular structure is defined by the substituents R1 to R5 according to the following table:

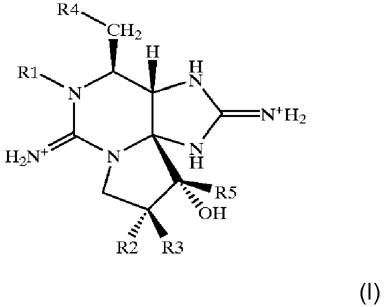

| **Compound** | **R1** | **R2** | **R3** | **R4** | **R5** |
|---|---|---|---|---|---|
| Saxitoxin | H | H | H | COONH₂ | OH |
| Neosaxitoxin | OH | H | H | COONH₂ | OH |
| Gonyaulatoxin 1 | OH | H | OSO⁻ ₃ | COONH₂ | OH |
| Gonyaulatoxin 2 | H | H | OSO⁻ ₃ | COONH₂ | OH |
| Gonyaulatoxin 3 | OH | OSO⁻ ₃ | H | COONH₂ | OH |
| Gonyaulatoxin 4 | H | OSO⁻ ₃ | H | COONH₂ | OH |
| Gonyaulatoxin 5 | H | H | H | COONHSO⁻ ₃ | OH |

The revelation that these alkaloids are produced by cyanobacteria is very recent. Only in the last 15 years it has been demonstrated that they are secondary metabolites found inside the cells, which under certain conditions can be released into the culture medium. Hence, in a continuous cyanobacterial culture, there are two sources of these compounds: one in the cell pellet and the second in the culture medium where cyanobacteria are cultured.

For the industrial production of these phycotoxins and their subsequent purification, a massive culture of these cyanobacteria is absolutely required, as that indicated in the Patent Application CL 722-2009. Before the process described in the Patent Application CL 722-2009, the development of large-volume industrial processes to obtain phycotoxins has not been achieved. However, phycotoxins were purified in basic research laboratories from shellfish contaminated with red tide, with the object of getting a few micrograms of these toxins to be used as analytical standards (standards to be used as reference compounds in chemical analyses).

To this date, no publication exists describing purification processes for these phycotoxins from contaminated shellfish or from dinoflagellates at industrial levels; in this case, industrial levels are considered to be in an order of magnitude of grams for the production of the metabolite or phycotoxin. Nothing has been also published about isolation of phycotoxins from cyanobacteria, which is not unexpected when considering that the cyanobacteria that are able to produce these phycotoxins have been only recently described in the literature (Lagos, 2003).

In this patent application, a purification and industrial production process for neosaxitoxin, saxitoxin and gonyaulatoxins 2/3 is presented, which uses an innovative biotechnological process starting from isolated cloned cyanobacteria that optimizes the continuous industrial production of these phycotoxins under controlled conditions, while keeping their potent biological activity during all the industrial process to produce an industrial active principle useful for the development of new drugs.

The integration of the production proposal and the technologies used in this invention considers the formerly described productive scaling of cyanobacteria to produce the necessary amount for new massive pharmacological uses and the development of new medicines or active principles for cosmetic applications.

The advantages of the present invention to massively produce these phycotoxins in controlled conditions from cyanobacteria are:
- The availability of clones from selected cyanobacterial strains able to produce phycotoxins with a unique composition and simple toxin profiles.
- Easy high-yield purification process for phycotoxins.
- Very favorable cost - production - yield relation that has not been achieved until now.

### Application field for the massive production of the phycotoxins neosaxitoxin, saxitoxin and gonyaulatoxins

The application field of these alkaloids includes their clinical applications as therapeutic agents in pathologies associated to muscular hyperactivity, such as muscular spasms and focal dystonias. Additionally, they can be used as local anesthetics in different broad-spectrum pharmaceutical preparations, which allows the classification of these phycotoxins as very useful products for the treatment of diverse pathologies, many of them with a large commercial demand. On the other hand, they can also be applied in cosmetic products against wrinkles and expression lines, which are applications with an obvious commercial demand. However, these phycotoxins are not commercially available in industrial amounts at the presently required levels. Hence the present purification, fractioning and production method for neosaxitoxin, saxitoxin and gonyaulatoxins solve a biotechnological problem that remained unsolved until now. The innovation proposed here allows the satisfaction of the large demand for the active principle of these pure phycotoxins in the national and international markets, at large scale for therapeutic and cosmetic uses and for other products to improve people's life quality.

These phycotoxins have very favorable physicochemical properties to be applied in the medicine and cosmetic industry. Their physical and chemical properties are the following: they are water-soluble, stable at room temperature, highly resistant to acid media and extreme temperatures (over 100°C), very low molecular weight (298-445 g/mol), which allows more easy, less dangerous and painless applications with no allergic or immune responses, unlike other compounds, such as the high-molecular weight botulinic toxic protein (Botox^{®}).

Due to these physical and chemical properties of phycotoxins, such as their low molecular weight and high chemical stability, it is possible to devise applications and biotechnological developments for many products and pharmaceutical preparations, such as uses in creams, gels, transdermal applications with ultrasound, infrared and other apparatuses, controlled release patches (patches adhered to the skin with continuous slow release). All these applications are possible due to the large stability of these compounds and the additional possibility of allowing chemical reactions with covalent bonding to other chemical compounds.

These advantages makes the phycotoxins neosaxitoxin, saxitoxin and gonyaulatoxins superior alternatives to the botulinic toxin, a compound that is widely used in dermocosmetic applications, which is a protease that has the disadvantages of being unstable, having a high molecular weight, generating an allergic immune response, producing structural damage (digesting nerve terminations and surrounding terminals, which causes adverse undesired effects that are currently very questioned worldwide), and self-digesting in short times.

The recently published clinical applications for the phycotoxins neosaxitoxin, saxitoxin and gonyaulatoxins are opening markets for massive demand applications: in the cosmetic field, for back pain, migraines, anal fissures, pain control in laparoscopic surgery and general neuropathic pain control, with millions of potential patients, which poses an incalculable future demand for these compounds.

Table 1 shows the advantages of the phycotoxins neosaxitoxin, saxitoxin and gonyaulatoxins when compared, for example, to the botulinic toxin (Botox^{®}), which produces a similar physiological response and therefore has similar applications to phycotoxins in clinical therapy and cosmetics, although the molecular mechanisms of action of phycotoxins and the botulinic toxin are very different.

**TABLE 1: Advantages of phycotoxins in comparison to the botulinic toxin**

| **Properties** | **Botox**^{®} | **Phycotoxins** |
|---|---|---|
| Active toxin | Botulinic toxin | neoSTX*; STX*; GTX* |
| Molecular weight | 900.000 | Ranging from 298 to 450 |
| Chemical stability | Unstable | Very stable |
| Storage | Frozen | Room temperature |
| Mechanism of action | Inhibition of the release of ACH* | Inhibition of the nervous impulse, inhibitor of the release of any neurotransmitter |
| Activation time | 5-15 days | Immediate (minutes) |
| Duration | 2 to 4 months | Dose-dependent |
| Topical application and other application modes | Not possible | Creams, gels, patches, iontophoresis, ultrasound. |

| | | |
|---|---|---|
| neoSTX: neosaxitoxin STX: saxitoxin GTX: gonyaulatoxin ACH: acetylcholine | | |

The relative easiness for the change of Botox^{®} users to the use of potential phycotoxin-derived products (including neosaxitoxin, saxitoxin and gonyaulatoxins) due to painless, lower-cost and instantaneous-effect applications generate competitive advantages for phycotoxins against Botox^{®}.

### OBJECT OF THE INVENTION

The general object of this invention is the industrial extraction, purification and production of biologically-active phycotoxins, compounds and secondary metabolites produced by cyanobacteria that have a high added and commercial value and are not commercially available at industrial production levels. The surprising fact about this invention is its ability to obtain these biologically active compounds along all the industrial process, which is a unique and surprising biotechnological process by itself.

A specific object of the present invention is a massive industrial high-yield purification process for the production of the phycotoxins neosaxitoxin, saxitoxin and gonyaulatoxins in a biologically active form, from practically unlimited levels of cyanobacteria produced in continuous cultures under controlled conditions.

Another specific object of the invention is to achieve a procedure to purify phycotoxins from wet and/or frozen cyanobacterial pellets, removing pigments and other main secondary metabolites that accompany the phycotoxins during the purification process.

### DESCRIPTION OF THE DRAWINGS

Figure 1 shows a chromatogram of 10 microliters of an extract of the cyanobacterium *Aphanizomenon* gracile that were injected into an analytical high-performance liquid chromatography (HPLC) equipment. The detection method used was fluorescence online detection. Peak 1, RT = 2.640 minutes, cyanobacterial pigments. Peak 2, RT = 7.980 minutes, neosaxitoxin. Peak 3, RT = 11.970 minutes, saxitoxin.
Figure 2 is a chromatogram of 10 microliters of a purified extract from *Aphanizomenon* gracile purified by preparative size exclusion high-performance liquid chromatography (separation by molecular size). The detection method used was fluorescence online detection. Peak 1, RT = 3.067 minutes, cyanobacterial pigments. Peak 2, RT = 9.373 minutes, neosaxitoxin. Peak 3, RT = 12.953 minutes, saxitoxin.
Figure 3 shows a chromatogram of an analytical standard including neosaxitoxin and saxitoxin as its mains components, with a small amount of a mixture of gonyaulatoxins (NL2 standard), determined and quantified by analytical high-performance liquid chromatography with online fluorescence detection. Peak 1, RT = 3.653 minutes, mixture of gonyaulatoxins; peak 2, RT = 5.040 minutes, neosaxitoxin; peak 3, RT = 7.440 minutes, saxitoxin.
Figure 4 is a chromatogram of a neosaxitoxin sample purified from extracts of *Aphanizomenon* gracile cyanobacteria (RT = 4.933 minutes). This corresponds to the fraction eluted from a preparative high-performance liquid chromatography using ion exchange columns with online fluorescence detection.
Figure 5 shows a chromatogram of analytical standards of gonyaulatoxins. From left to right: Peak 1: GTX4 (gonyaulatoxin 4); Peak 2: GTX1 (gonyaulatoxin 1); Peak 3: GTX5 (gonyaulatoxin 5); Peak 4: GTX3 (gonyaulatoxin 3); Peak 5: GTX2 (gonyaulatoxin 2).
Figure 6 shows a chromatogram of a sample of extract from the cyanobacterium C. *raciborskii.* Peak 1, RT = 8.873 minutes, peak 2, RT = 10.927 minutes, peak 3, RT = 13.260 minutes; peak 4, RT = 16.647 minutes.
Figure 7 is a chromatogram of a fraction from *C. raciborkii* partially purified by size exclusion high-performance liquid chromatography, with a dominant presence of the epimers GTX 3 and GTX 2, respectively.
Figure 8 shows a chromatogram of the final pure fraction of the epimers GTX 3 and GTX 2 obtained from a continuous culture of *C. raciborkii* cyanobacteria.
Figure 9 shows an elution plot of a size exclusion preparative chromatography showing the obtained fractions and identifying the range of fractions where the different present compounds are present (organic compounds, phycotoxins and salts).
Figure 10 shows an elution plot of an anion exchange preparative chromatography showing the obtained fractions and identifying the range of fractions where the different present compounds are present (phycotoxins and salts).
Figure 11 shows an elution plot of a cation exchange preparative chromatography showing the obtained fractions and identifying the range of fractions where the different present compounds are present (phycotoxins and salts).
Figure 12 shows an elution plot of a size exclusion preparative chromatography showing the obtained fractions and identifying the range of fractions where the different purified phycotoxins are present (saxitoxin and neosaxitoxin).

### BRIEF DESCRIPTION OF THE INVENTION

This document describes the purification and industrial production of the phycotoxins neosaxitoxin, saxitoxin and gonyaulatoxins in a biologically active form from cultures of cyanobacteria able to produce these phycotoxins.
The cyanobacteria used in the present invention can belong to the genera: *Cylindrospermopsis sp, Microcystis sp, Anabaena sp, Gomphosphaeria sp, Oscillatoria sp, Aphanizomenon sp (Aphanizomenon issatchenkoi, Aphanizomenon flos-aquae, Aphanizomenon gracile).*

Cyanobacteria are preferentially obtained from a continuous massive semi-automatic culture under controlled conditions, such as that described in the Chilean Patent Application CL 722-2009, presented simultaneously with the present application by the same authors.

The industrial purification of the phycotoxins neosaxitoxin, saxitoxin and gonyaulatoxins from cyanobacteria in a continuous culture is carried out using a method that includes extraction, fractioning, solvent partitioning, partitioning on solid phases, and partitioning in liquid-solid interfaces of these phycotoxins (neosaxitoxin, saxitoxin and gonyaulatoxins) from culture cell pellets or from the culture supernatant. This is a continuous, sequential and semi-automatic process that produces industrial amounts of an active principle that keeps its biological activity throughout all the purification process until the final product. The process involves chemical and biochemical processes in several fractioning steps using differential centrifugation, extraction with aqueous and organic solvents, partitioning in hydrophobic solvent phases, partitioning on solid phases, column purification and several types of preparative high-performance chromatographies (cation exchange, anion exchange and size exclusion), to obtain partially purified extracts with no pigments, corresponding to fractions with simple phycotoxin profiles and pure phycotoxins from these fractions (Figures 1 to 8). All these steps are part of a unique biotechnological process and part of an industrial process that has been only developed in Chile.

### DETAILED DESCRIPTION OF THE INVENTION

The invention is related to a purification method for the industrial production of the phycotoxins neosaxitoxin, saxitoxin and gonyaulatoxins, keeping their biological activity throughout all the industrial purification process, from cyanobacteria able to produce these phycotoxins.

The cyanobacteria able to produce these phycotoxins belong to the genera: *Cylindrospermopsis sp., Microcystis sp., Anabaena sp., Gomphosphaeria sp., Oscillatoria sp*., *Aphanizomenon spp.* (*Aphanizomenon issatchenkoi, Aphanizomenon flos-aquae, Aphanizomenon gracile).*

The starting material is a unialgal culture of a cyanobacterial species; the unialgal culture is indispensable to obtain these purified phycotoxins in high yields. This is part of the growth and scaling process up to an industrial level to get a high amount of phycotoxins in the starting material.

Cyanobacteria are obtained through the development of a method and procedure of continuous massive semi-automatic culture under controlled conditions that sustains a permanent logarithmic growth of cyanobacteria, described in the Chilean Patent Application CL 722-2009, presented simultaneously with the present application by the same authors.

During the culture process of these cyanobacterial species, phycotoxins are accumulated inside the cells but are also released into the culture medium, therefore constituting two different phycotoxin sources.

For example, in the culture of *Aphanizomenon gracile,* the main component released from cyanobacterial cells is neosaxitoxin (Figures 2 and 4).

Therefore, there are two possible sources to obtain phycotoxins from an industrial cyanobacterial culture:
- from the filtered supernatants (with no cells or filaments) of the cyanobacterial growth medium;
- from the wet pellets obtained by centrifugation of the developing culture, comprising cyanobacterial cells and filaments.

The production of the target phycotoxins, which depend on the producer cyanobacteria cultured in each case, can be obtained for any of the mentioned cyanobacteria, from the supernatants or from the obtained pellets.

The industrial production of the phycotoxins neosaxitoxin, saxitoxin and gonyaulatoxins from a continuous culture of cyanobacteria is achieved using a method including extraction, fractioning, solvent partitioning, solid phase partitioning, and liquid-solid phase partitioning of these phycotoxins (neosaxitoxin, saxitoxin and gonyaulatoxins) from culture cells (pellet). The process involves chemical and biochemical processes in several fractioning steps using differential centrifugation, extraction with aqueous and organic solvents, partitioning in hydrophobic solvent phases, partitioning on solid phases, column purification and several types of preparative high-performance chromatographies (cation exchange, anion exchange and size exclusion), to obtain partially purified extracts with no pigments, corresponding to fractions with simple phycotoxin profiles and pure phycotoxins from these fractions (Figures 1 to 8). The fundamental approach is directed to the obtainment of a stable active principle that maintains all the potency of its biological activity in such a way as to be used as a basis for the development of new pharmaceutical products.

In a simplified way, the neosaxitoxin, saxitoxin and gonyaulatoxin purification method of the invention comprises the following steps:
a. providing a source of phycotoxins, namely a cyanobacterial clone culture, which is separated into a culture medium and a wet cyanobacterial cell pellet or a frozen cyanobacterial cell pellet;
b. said pellet is lysed using homogenization, solvent extraction, bead mill, freeze/thaw cycles, ultrasonication or enzymatic lysis;
c. the obtained material is subjected to a cold extraction and organic / aqueous phase separation, namely a 50% by volume of a 1:1 chloroform:methanol mixture and 50% by volume of 1 mM acetic acid, at pH between 4 and 5, and subsequently separating the aqueous and organic phases using chloroform:methanol 1:1 by volume, which is repeated between 1 and 5 times;
d. obtaining a concentrate of the aqueous phase obtained in step (c);
e. centrifuging the concentrated aqueous phase to obtain a supernatant;
f. passing the supernatant through a solid matrix of diatomaceous earth, where phycotoxins are retained, and washing with a washing solution; then, obtaining a phcotoxin eluate with an elution solution;
g. passing the eluate containing the phycotoxins through a matrix of activated charcoal, where again phycotoxins are retained , and then washing with distilled water to remove the retained pigments and impurities; phycotoxins must be eluted with an elution solution therefrom;
h. passing the eluate from the previous step that contains the phycotoxins again through a solid matrix of diatomaceous earth, washing and eluting once more with an extraction solution;
i. evaporating the organic components of the aqueous eluate of the previous step, to get a partially purified phycotoxin extract;
j. subjecting the partially purified phycotoxin extract obtained in the previous step to a biochemical separation and fractioning process by preparative HPLC (HPLC-Prep: preparative high-performance liquid chromatography) in several steps, according to the physical or chemical fractioning principle used, which can sequentially comprise: size exclusion, anion exchange, cation exchange and size exclusion again.

In this way, pure phycotoxin preparations are obtained, which are suitable for pharmaceutical and cosmetic applications.

The method of the present invention provide for the first time in the state of the art a procedure to purify phycotoxins, such as neosaxitoxin, saxitoxin and gonyaulatoxins, from cyanobacteria able to produce these phycotoxins.

This method comprises providing a suitable amount of a source of phycotoxins, *e.g*. a cyanobacterial culture, the culture medium wherein cyanobacteria grow in said culture, or a cyanobacterial cell pellet.

If a cyanobacterial culture is used, cells are separated from the culture medium, *e.g*. using a centrifugation step. In this way, a liquid aqueous phase corresponding to the culture medium and a wet pellet corresponding to the cyanobacterial cell are obtained. Cyanobacterial pellets can be frozen of directly processed, since the freezing step does not affect the subsequent purification of the phycotoxins neosaxitoxin, saxitoxin and gonyaulatoxins.

The pellets are lysed using suitable methods known in the state of the art, such as homogenization, solvent extraction (organic phase / aqueous phase), bead mill, freeze/thaw cycles, ultrasonication, enzymatic lysis, and the like.

The material obtained from the lysis of cyanobacteria is subjected to a cold extraction using a mixture of organic solvents in an aqueous phase (chloroform:methanol 1:1 with 10 mM acetic acid comprising 50% by volume of the previous mixture), and subsequently separating the organic and aqueous phases using an organic phase at pH 5 comprising chloroform:methanol 1:1 by volume, which is repeated between 1 and 5 times. All the aqueous phases are collected to form a single aqueous phase with which the purification procedure is continued.

The aqueous phase obtained in the previous step or the culture medium supernatant is concentrated using, for example, a rotary evaporator at room temperature until reaching a concentration between 5 to 20 times that of the original volume.
This concentrate is centrifuged at a relative centrifugal force ranging from 15,000 × g to 25,000 × g, for periods ranging from 10 to 40 minutes.
The centrifugate supernatant is passed through a diatomaceous earth column, washing the column with 5 to 15 times its volume of a suitable solution, *e.g*. 50 mM acetic acid. The phycotoxins are eluted with a suitable solution, *e.g*. an alcoholic extraction mixture, ethanol:water:5 mM acetic acid in a 2:1:1 ratio (vol/vol/vol).
The eluate from the previous step is passed through activated charcoal columns, which are then washed with distilled water to remove the retained pigments and impurities. The phycotoxins are retained in the column and are eluted with a suitable solution, *e.g*. an alcoholic elution mixture, ethanol:water:1 mM acetic acid in a 3:2:1 ratio (vol/vol/vol) at pH 5. The eluate from the previous step is passed again through a diatomaceous earth column. The columns are washed with a suitable solution of 50 mM acetic acid using a volume equivalent to 10 times the volume of the matrix and phycotoxins are eluted with an appropriate solution, *e.g*. by solvent extraction with a suitable alcoholic solution at pH 5, for example a 1:1:1 (vol/vol/vol) chloroform/methanol/water mixture.
The eluate from the previous step is left in an aqueous phase evaporating the organic solvents, *e.g*. using a "speed vac" equipment (Savant, NY, USA). A partially purified phycotoxin extract is thus obtained.

The partially purified extract from the previous step is subjected to a preparative HPLC (preparative high-performance liquid chromatography) in several steps that can sequentially comprise: size exclusion, anion exchange, cation exchange and size exclusion again. Carrying out several successive chromatographies guarantees an analytical purity suitable for the requirements of the pharmaceutical industry. However, in certain cases, depending on the required purity degree, phycotoxins can be purified using only one HPLC-Prep step.

It must be understood that the values mentioned for the ratios and proportions of the ingredients of each solution, ingredient concentrations or pH values can vary in a range of ±5% without altering the result of the invention.

In a more detailed and schematic way, the phycotoxin purification method of the invention can be defined by the following steps:
a. A suitable amount of a phycotoxin source is provided, such as the culture of a pure cyanobacterium, according to the disclosure of the Chilean application "Culture of cyanobacteria able to produce neosaxitoxin and saxitoxin at industrial levels" (CL 722-2009), presented simultaneously with the present application by the same authors.
   - Separation of the cells and the culture medium by centrifugation. 1 L culture volumes obtained from each reactor are centrifuged at 10,000 × g for 25 minutes, to obtain a supernatant and a precipitate of wet cyanobacterial pellet.
   - Optional step to freeze the cell pellets. Cell pellets can be frozen to be purified afterwards or the purification can be immediately started.
b. The pellet is lysed using a preferred pellet lysis method and then is weighed and the same volume of the mixture of organic solvents in aqueous phase by weight of pellet (chloroform:methanol 1:1 + 10 mM acetic acid in a 50% mixture with the former mixture) is added to destroy the cell wall and the cytoplasmic membrane. This is done in a slightly acid medium (pH 5.0). A slightly acid medium is a unique and important requirement that allows the active principle to remain active.
c. The material from the cell lysis is subjected to a cold extraction and phase separation (organic / aqueous).
   - The extraction of the organic phase previously obtained is repeated.
   - The two organic phases obtained are combined.
   - The aqueous phase is extracted twice with organic phase at pH 5.0. The same volume of a 1:1 vol/vol chloroform:methanol solution is used.
d. The extracted aqueous phase is concentrated 10 times by volume (getting one tenth of the original volume), *e.g*. using a rotary evaporator at room temperature.
e. The concentrate is centrifuged, preferably at 20,000 × g for 30 minutes.
f. The supernatant aqueous phase from the centrifugation step is treated with a solid diatomaceous earth matrix in a step consisting in passing the supernatant through a diatomaceous earth column. The column is washed with 50 mM acetic acid with an amount of preferably 10 times the volume of the column, and the phycotoxin retained in the column is extracted with an extraction alcoholic solution or elution buffer (2:1:1 vol/vol/vol ethanol:water:5 mM acetic acid).
g. The eluate from the column of the previous step is now passed through activated charcoal columns, which are then washed with distilled water to remove the retained pigments and impurities. The phycotoxins are retained in the columns, which are eluted with an alcoholic elution mixture (3:2:1 vol/vol/vol 7ethanol:water:1 mM acetic acid, pH 5.0). This fractioning removes a large fraction of the more abundant pigments and low-molecular weight molecules present in the cyanobacterial lysate, such as amino acids, nucleotide bases, peptides, sugars (mono and disaccharides).
h. A new differential elution from solid diatomaceous earth matrixes is carried out subsequently. Diatomaceous earth columns are charged with the extract eluted from the activated charcoal. Again the phycotoxins are retained on the solid matrix by hydrophobic interaction effects. These columns are initially washed to elute a large fraction of the low-molecular weight components, which are eliminated. The retained phycotoxins are subsequently eluted from the column by solvent extraction with a 1:1:1 vol/vol/vol chloroform:methanol:water mixture. The use of diatomaceous earth columns generates a total stratified fractioning. This massive procedure for large amounts does not generate the fractioning achieved with these columns when used in a batch technique. Another surprising effect is represented by the substitution of the batch technique by a column separation, which unexpectedly produces a large initial pre-purification of the massive industrial material, with a consequent save in costs and a large increase in efficiency.
i. The eluate from the second diatomaceous earth column is left in an aqueous phase evaporating the organic solvents, *e.g*. using a "speed vac" equipment (Savant, NY, USA). A partially purified phycotoxin extract is thus obtained.
j. This partially purified extract is subjected to a preparative HPLC (HPLC-Prep: preparative high-performance liquid chromatography).
   A) Separation by size preparative HPLC): For this separation, high-pressure stainless steel columns filled with Bio-Gel P-2 (Bio-Rad) Fine, 45-90 microns (wet) are used. These columns have 10 cm in diameter and 85 centimeters in length, and are packed under pressure with dry Bio-Gel P-2 resin, which have an exclusion size of 1800 Daltons. This resin separates, in the void volume (Vo), any molecule with a size larger or with a molecular weight higher than 1800 Daltons. In this way, all macromolecules and even small 10-amino acid peptides pass in the initial fractions (at the run front), while lower-molecular weight compounds, such as the phycotoxins (298 to 445 Daltons) are retained. Using this size exclusion resin, 90% of the undesired organic material is eliminated, including a large amount of pigments that are characteristic of these cyanobacteria. All these compounds and the soluble macromolecules exit the column in the first fractions (first 5 liters eluted). The intermediate fractions that are near to the end fractions are those containing phycotoxins. In the final fractions, a large part of the salt present in the extracts is eliminated. The use of this resin for size exclusion is a completely innovative concept, especially in the form used herein. The fractioning columns are a particular design of this invention not only in their size but also in the way the columns are filled. This purification step is transcendental, efficient and practically delivers a partially purified active principle. All the preparative liquid chromatography fractionings used from here on use this innovative biotechnological process to fill the column with a solid matrix at a high pressure, which generates practically unlimited contact surfaces that are totally suitable for industrial purification processes of massive active principles. In these preparative HPLC processes, it is also worth mentioning the favorable celerity of the process, which is a fundamental issue to achieve a pure biologically active compound. Figure 9 shows an elution plot of a preparative chromatography that exemplifies this size exclusion step. The phycotoxin-containing fractions are concentrated and then subjected to a second separation corresponding to an anion exchange process. Size exclusion is also useful to remove most of the salts present in these extracts, which were not completely removed in the phase fractionings initially carried out in this purification process. This is important, since these salts could interfere with the ion exchange processes. Therefore, not only macromolecules and molecules with a molecular weight over 1800 Daltons are removed in this separation, but most of the low-molecular weight salt components (sodium 23, chlorine 35, etc.) are also removed.
   B) **Anion exchange:** Columns similar to preparative size exclusion columns are assembled, but now using an anion exchange resin. Here, Cellex-D (Bio-Rad) resin, with an exchange capacity of 0.66 milliequivalents per gram, is used. In this preparative chromatography, phycotoxins with a positive net charge (+2 and +1) at neutral pH, elute in the first fractions (initial 2.5 liters), since they are not retained in the column because of their positive charge. Hence, these phycotoxins elute in the column void volume. In these columns, only compounds with negative charge are retained and they are eluted at the end of the chromatography in fractions that do not contain phycotoxins and are therefore eliminated. The initial fraction that contains the total amount of phycotoxins is concentrated again (industrial freeze-drier) and then applied into preparative cation exchange columns. This fraction contains substantially pure phycotoxins with a net charge of +2 (neosaxitoxin and saxitoxin) and +1 (gonyaulatoxins).
   C) **Cation exchange:** In this preparative chromatography (using columns with the same dimensions as the previous ones, filled with Bio-Rex^{®} 70 from Bio-Rad), phycotoxins will be retained, which will be separated in two major groups: those with a +2 net charge and those with +1 net charge. In the initial fractions, the remaining small fraction of salts with a negative charge (measured by electrical conductivity), then gonyaulatoxins are eluted, and finally saxitoxin and neosaxitoxin elute. These last two separated phycotoxins are separated, and a fraction containing a mixture of both of them with a proportion of 2/3 neosaxitoxin and 1/3 saxitoxin. The last eluting fraction contains substantially pure neosaxitoxin.
   D) Finally, several concentrated final fractions from the cation exchange separation are separated again in the size exclusion column Bio-Gel P-2 (using an identical process to the one described in A), to remove the remaining salts and obtain pure saxitoxin eluted in distilled water.

It must be understood that the values mentioned for the ratios and proportions of the ingredients of each solution, ingredient concentrations or pH values can vary in a range of ±5% without altering the result of the invention.

The phycotoxins suitable to be purified with the method described above are neosaxitoxin, saxitoxin and gonyaulatoxins. The obtainment of one particular toxin will depend on the cyanobacterial strain from which purification is carried out, because certain strains preferentially produce on particular type of phycotoxin. The characteristic retention times of the target compounds (phycotoxins) are considered as separation and fractioning criteria, as presented in the following examples of the present application.

The purity of each stock (batch) can be determined by visible and ultraviolet spectroscopy, and also using mass spectrometry. The final detection and quantification can be carried out by spectrofluorometry. This last analytical method for quantitative detection is specific for all phycotoxins, such as neosaxitoxin, saxitoxin and gonyaulatoxins (Lagos, 1998. Microalgal blooms: a global issue with negative impact in Chile. Biol. Res. 31: 375-386).

### EXAMPLES

### Example 1: Phycotoxins obtained and purified from Aphanizomenon gracile maintained in continuous cultures under controlled conditions.

As indicated before, in *Aphanizomenon gracile* cultures using a selected single clone, neosaxitoxin is the main phycotoxin produced. In this particular case, this clone also produces a lower amount of saxitoxin.

The production of neosaxitoxin from *Aphanizomenon gracile* cyanobacteria was carried out from an initial amount of 10 milligrams of wet *Aphanizomenon gracile* cells. The pellet (obtained in step a) was subjected to the method described above, *i.e*.:
b. for 10 mg of cell pellet, 10 mL of the same volume in weight of the mixture of organic solvents in aqueous phase (1:1 chloroform:methanol + 50 mM acetic acid in a 50% proportion with the previous mixture) were added to destroy the cell wall and the cytoplasmic membrane, in a slightly acid medium (pH 5.0).

The lysed cells from step b are subjected to a cold extraction and phase separation (organic / aqueous).
d. The extracted aqueous phase from step c is concentrated 10 times by volume (getting one tenth of the original volume), *e.g*. using a rotary evaporator at room temperature.
e. The concentrate is centrifuged at 20,000 × g for 30 minutes.
f. 2 mL of the supernatant from the centrifugation are passed through a diatomaceous earth column. This column is washed with 10 times its volume (20 ml in this case) of 50 mM acetic acid. After the washing step, neosaxitoxin retained in the column is extracted with 5 mL of an alcoholic extraction mixture (2:1:1 vol/vol/vol ethanol:water:5 mM acetic acid).
g. The eluate from the column obtained in step f is passed through activated charcoal columns, which are then washed with distilled water to remove the retained pigments and impurities. Phycotoxins remain retained in the columns, and are eluted with 10 mL of an alcoholic elution mixture (3:2:1 vol/vol/vol ethanol:water:1 mM acetic acid) at pH 5.
h. The eluate is passed through a diatomaceous earth column and washed with 50 mM acetic acid. Subsequently, the toxin is eluted from the column with 5 mL of a 1:1:1 vol/vol/vol chloroform:methanol:water mixture.
i. The eluate from the second diatomaceous earth column is left in an aqueous phase evaporating the organic solvents using a "speed vac" equipment (Savant, NY, USA). A partially purified neosaxitoxin extract is obtained.
j. The partially purified extract is subjected to a size exclusion preparative HPLC (preparative high-performance liquid chromatography).

Each reactor after 2 culture days produces an average of 652,4 micrograms of total toxin per each collected liter. The average neosaxitoxin/saxitoxin ratio is 8.47. In percentages, an average of 11.8% saxitoxin and 88.2% neosaxitoxin is obtained. The main objective is neosaxitoxin production, which is sought, heightened and protected since this compound is the active principle patented in clinical applications as a drug or cosmetic. Neosaxitoxin has a biological effect that is 25% more potent than saxitoxin, and is also the most potent phycotoxin described so far.

Figures 1, 2 and 4 show chromatograms of HPLC runs detected by online fluorescence detection that describe a phycotoxin profile produced by the species *Aphanizomenon gracile.* Figures 1 and 2 describe HPLC profiles of fractions presenting several degrees of purification from *Aphanizomenon gracile* extracts. Once again, it is worth mentioning that those are chromatograms from analytical high-performance liquid chromatographies that are carried out to detect and quantify phycotoxins. Those are not preparative chromatograms from the industrial process. These chromatograms are only useful to show the degree of purity and the content of each purified fraction.

Figure 1 shows a chromatogram of an unpurified *Aphanizomenon gracile* extract carried out to know and quantify the profile of phycotoxins present in this original extract. The chromatogram shows a simple profile with a major proportion of neosaxitoxin and a lower amount of saxitoxin (less than 13%).

Figure 2 shows the purification of 10 microliters of an extract from *Aphanizomenon gracile* grown in continuous culture under controlled conditions, partially purified by preparative size exclusion high-performance liquid chromatography (separation by molecular size). In this partially purified extract, a typical peak at RT = 9.373 minutes that saturates the chromatogram, which corresponds to neosaxitoxin, is already present, indicating the presence of a large amount of neosaxitoxin in this fraction.

Figure 4 shows a neosaxitoxin sample purified from extracts of *Aphanizomenon* gracile cyanobacteria (RT = 4.933 minutes) that is obtained in the fraction eluted from HPLC with ion exchange columns, corresponding the last purification step (final step of the method described above). A single peak is obtained, corresponding to a single component, which is pure neosaxitoxin in this case.

**TABLE 2: Production of neosaxitoxin and saxitoxin as a function of the number of filaments and the wet weight of the Aphanizomenon gracile pellet.**

| **neoSTX mM** | **STX mM** | **neoSTX µg/ml** | **STX µg/ml** | **neoSTX pg/fil.** | **STX pg/fil.** | **Cyano. fil./ml*** |
|---|---|---|---|---|---|---|
| 8.98 | 3.50 | 2.83 | 1.05 | 2.42 | 0.90 | 1,170,000.00 |
| 13.66 | 4.91 | 4.30 | 1.47 | 3.44 | 1.18 | 1,250,000.00 |
| 3.42 | 1.03 | 1.08 | 0.31 | 1.71 | 0.49 | 630,000.00 |
| 17.82 | 4.78 | 5.61 | 1.43 | 9.42 | 2.41 | 596,000.00 |
| 17.81 | 4.02 | 5.61 | 1.21 | 13.17 | 2.83 | 426,000.00 |
| 7.66 | 1.47 | 2.41 | 0.44 | 4.94 | 0.91 | 488,000.00 |
| 10.87 | 2.19 | 3.42 | 0.66 | 10.25 | 1.97 | 334,000.00 |
| 10.52 | 2.07 | 3.31 | 0.62 | 4.38 | 0.82 | 756,000.00 |
| 10.77 | 1.96 | 3.39 | 0.59 | 7.47 | 1.30 | 454,000.00 |
| 17.24 | 2.99 | 5.43 | 0.90 | 13.12 | 2.16 | 414,000.00 |
| 5.31 | 0.41 | 1.67 | 0.12 | 5.19 | 0.38 | 322,000.00 |
| 12.26 | 2.17 | 3.86 | 0.65 | 9.47 | 1.59 | 408,000.00 |
| 9.84 | 1.94 | 3.10 | 0.58 | 17.42 | 3.27 | 178,000.00 |

| | | | | | | |
|---|---|---|---|---|---|---|
| STX: saxitoxin neoSTX: neosaxitoxin Cyano. fil.: cyanobacterial filaments * Cyanobacterial filaments are associations of 20 to 100 cyanobacterial cells. These cyanobacteria form filaments in solution and those filaments are counted using the magnification of an inverted microscope. | | | | | | |

The yields obtained represent a surprising unexpected production of pure phycotoxins (neosaxitoxin and saxitoxin) per milligram of wet cyanobacteria, when compared to the production of filaments and cyanobacterial pellets in usual culture conditions described so far in small culture flasks without continuous micro-aeration and with light:day and no light:night cycles.

In the example described herein, cyanobacteria are always in the logarithmic growth phase, with permanent illumination 24 hours a day and with permanent collection of cyanobacteria, inducing permanent growth by adding new nutrients in a volume that is equivalent to the volume collected in each harvest.

### Example 2: Phycotoxins obtained and purified from Cylindrospermopsis raciborskii maintained in continuous cultures under controlled conditions.

The cyanobacterium *Cylindrospermopsis raciborskii* was cultures according to the continuous massive semi-automatic culture method under controlled conditions in permanent logarithmic growth, described in the Chilean Patent Application presented simultaneously with the present application by the same authors.

The unpurified extract of the strain *Cylindrospermopsis raciborskii* has a chromatographic profile according to Figure 6. *Cylindrospermopsis raciborskii* has a toxin profile where GTX 3 and GTX 2 are predominant, with the longest retention times in the column (the last two peaks at right, respectively).

The *Cylindrospermopsis raciborskii* extract from the continuous culture is subjected to the purification process corresponding to the steps mentioned above, in the same conditions described for Example 1, and already at the size exclusion preparative HPLC step, the GTX3 and GTX2 epimers are almost exclusively obtained, as shown in Figure 7.

The final fraction of the phycotoxin purification method from *Cylindrospermopsis raciborskii* has only GTX3 and GTX2, as shown in Figure 8 and fractions containing each gonyaulatoxin can be collected to obtain large amounts of pure GTX3 and GTX2.

## Claims

1. Method for the industrial purification of the biologically active phycotoxins neosaxitoxin, saxitoxin and gonyaulatoxins from cyanobacteria, said method comprising the steps of:
a) providing a as a source of phycotoxins, namely a cyanobacterial clone culture, which is separated into a culture medium and a wet cyanobacterial cell pellet or a frozen cyanobacterial cell pellet;
b) said pellet is lysed using homogenization, solvent extraction, bead mill, freeze/thaw cycles, ultrasonication or enzymatic lysis;
c) the material from the cyanobacterial cell lysis obtained in step b) is subjected to a cold extraction and organic / aqueous phase separation, namely a 50% by volume of a 1:1 chloroform:methanol mixture and 50% by volume of 1 mM acetic acid, at pH between 4 and 5, and subsequently separating the organic and aqueous phases using chloroform:methanol 1:1 by volumen, which is repeated between 1 and 5 times;
d) obtaining a concentrate of the aqueous phase obtained in step (c);
e) centrifuging the concentrate obtained in step d) to obtain a supernatant;
f) passing the supernatant through a diatomaceous earth column, and washing the column with a washing solution; then obtaining a phycotoxin eluate with an elution solution;
g) the eluate from the previous step is passed through activated charcoal columns, and said activated charcoal columns are then washed with distilled water to remove the retained pigments and impurities; phycotoxins are eluted with an elution solution;
h) the eluate from the previous step is passed again through a diatomaceous earth column; the columns are washed with a solution as per in step f), and phycotoxins are eluted with an extraction solution;
i) the eluate from the previous step is left in an aqueous phase evaporating the organic solvents to obtain a partially purified phycotoxin extract;
j) The partially purified extract from the previous step is subjected to a preparative high-performance liquid chromatography in several steps to obtain the pure biologically active phycotoxin.

2. Method according to claim 1, in which the extraction of step (c) is repeated 3 times.

3. Method according to claim 1, in which the washing solution of steps f) and h) is 50 mM acetic acid, in step (f) the columns is washed with 5 to 15 times its volume and the elution solution is an alcoholic extraction mixture.

4. Method according to claim 1, in which the elution solution of step g) is an alcoholic elution mixture.

5. Method according to claim 1, in which the solution of step h) is a 1:1:1 vol/vol/vol chloroform:methanol:water mixture.

6. Method according to claim 1, in which the chromatographic steps of step j) are sequentially size exclusion, anion exchange, cation exchange and size exclusion.

7. Method according to any one of claims 1 to 6, in which the cyanobacteria able to produce phycotoxins belong to the genera *Cylindrospermopsis sp, Microcystis sp, Anabaena sp, Gomphosphaeria sp, Oscillatoria sp, Aphanizomenon sp* and *Lyngbya wollei.*

## Patentansprüche

1. Verfahren zur industriellen Reinigung der biologisch aktiven Phycotoxine Neosaxitoxin, Saxitoxin und Gonyaulatoxine aus Cyanobakterien, wobei das genannte Verfahren folgende Schritte umfasst:
a) das Bereitstellen einer Phycotoxinquelle, nämlich einer cyanobakteriellen Klonkultur, welche in ein Kulturmedium und einen feuchten cyanobakteriellen Zellniederschlag oder einen gefrorenen cyanobakteriellen Zellniederschlag getrennt wird;
b) das Lysieren des genannten Niederschlags unter Verwendung von Homogenisierung, Lösungsmittelextraktion, Perlmühle, Gefrier/Auftau-Zyklen, Ultraschallbehandlung oder enzymatischer Lyse;
c) das Aussetzen des Materials aus der Lyse von cyanobakteriellen Zellen, welches in Schritt b) erhalten wird, einer Kaltextraktion und einer Trennung in organische/wässrige Phasen, nämlich 50 Vol.-% eines 1:1 Chloroform:Methanol-Gemisches und 50 Vol.-% 1 mM Essigsäure, bei einem pH-Wert zwischen 4 und 5, und das anschließende Trennen der organischen und der wässrigen Phase unter Verwendung von 1:1 Vol/Vol Chloroform:Methanol, was zwischen einmal und fünfmal wiederholt wird;
d) das Erhalten eines Konzentrats aus der in Schritt c) erhaltenen wässrigen Phase;
e) die Zentrifugierung des in Schritt d) erhaltenen Konzentrats um einen Überstand zu erhalten;
f) das Passieren des Überstands durch eine Kieselgursäule, und das Waschen der Säule mit einer Waschlösung; dann das Erhalten eines Phycotoxineluats mit einer Elutionslösung;
g) das Passieren des Eluats aus dem vorhergehenden Schritt durch Aktivkohlesäulen, und dann das Waschen der genannten Aktivkohlesäulen mit destilliertem Wasser, um die zurückgehaltenen Pigmente und Verunreinigungen zu beseitigen; das Eluieren der Phycotoxine mit einer Elutionslösung;
h) das erneute Passieren des Eluats aus dem vorhergehenden Schritt durch eine Kieselgursäule; das Waschen der Säulen mit einer Lösung gemäß Schritt f), und das Eluieren der Phycotoxine mit einer Extraktionslösung;
i) das Lassen des Eluats aus dem vorhergehenden Schritt in einer wässrigen Phase unter Verdampfung der organischen Lösungsmittel, um einen teilweise gereinigten Phycotoxinextrakt zu erhalten;
j) das Aussetzen des teilweise gereinigten Extrakts aus dem vorhergehenden Schritt einer präparativen Hochleistungsflüssigkeitschromatographie in mehreren Schritten, um das reine biologisch aktive Phycotoxin zu erhalten.

2. Verfahren nach Anspruch 1, in welchem die Extraktion aus Schritt (c) dreimal wiederholt wird.

3. Verfahren nach Anspruch 1, in welchem die Waschlösung aus den Schritten f) und h) 50 mM Essigsäure ist, in Schritt (f) die Säulen mit dem 5- bis 15-fache ihres Volumens gewaschen werden und die Elutionslösung ein alkoholisches Extraktionsgemisch ist.

4. Verfahren nach Anspruch 1, in welchem die Elutionslösung aus Schritt g) ein alkoholisches Elutionsgemisch ist.

5. Verfahren nach Anspruch 1, in welchem die Elutionslösung aus Schritt h) ein 1:1:1 Vol/Vol/Vol Chloroform:Methanol:Wasser-Gemisch ist.

6. Verfahren nach Anspruch 1, in welchem die chromatographischen Schritte aus Schritt j) sequenziell Größenausschluss, Anionenaustausch, Kationenaustausch und Größenausschluss sind.

7. Verfahren nach einem der Ansprüche 1 bis 6, in welchem die Cyanobakterien, die in der Lage sind, Phycotoxine zu produzieren, zu den Gattungen *Cylindrospermopsis sp, Microcystis sp, Anabaena sp, Gomphosphaeria sp, Oscillatoria sp*, *Aphanizomenon sp* und *Lyngbya wollei* gehören.

## Revendications

1. Procédé de purification industrielle de phycotoxines biologiquement actives neosaxitoxine, saxitoxine et gonyaulatoxines de cyanobactéries, ledit procédé comprenant les étapes de :
a) fournir en tant que source de phycotoxines, à savoir une culture de clones cyanobactériens, qui est séparé en un milieu de culture et un culot de cellules cyanobactériennes humides ou un culot de cellules cyanobactériennes congelées ;
b) lyser ledit culot au moyen d'homogénéisation, extraction par solvant, broyeur à billes, cycles de congélation/décongélation, ultrasonication ou lyse enzymatique ;
c) soumettre la matière de la lyse de cellules cyanobactériennes obtenu dans l'étape b) à une extraction à froid, et une séparation en phase organique/aqueuse, à savoir 50% en volume d'un mélange chloroforme:méthanol 1:1 et 50% en volume d'acide acétique 1 mM, à un pH entre 4 et 5, et ensuite séparer les phases organique et aqueuse en utilisant du chloroforme:méthanol 1:1 en volume, ce qui est répété entre 1 et 5 fois ;
d) obtenir un concentré de la phase aqueuse obtenue dans l'étape c) ;
e) centrifuger le concentré obtenu dans l'étape d) afin d'obtenir un surnageant ;
f) faire passer le surnageant au travers d'une colonne de terres à diatomées, et laver la colonne avec une solution de lavage ; ensuite obtenir un éluat de la phycotoxine avec une solution d'élution ;
g) faire passer l'éluat de l'étape précédente au travers de colonnes de charbon actif, et ensuite laver lesdites colonnes de charbon actif avec de l'eau distillée pour enlever les pigments et les impuretés retenus ; éluer les phycotoxines avec une solution d'élution ;
h) faire passer à nouveau l'éluat de l'étape précédente au travers d'une colonne de terres à diatomées ; laver les colonnes avec une solution comme pour l'étape f), et éluer les phycotoxines avec une solution d'extraction ;
i) laisser l'éluat de l'étape précédente dans une phase aqueuse en évaporant les solvants organiques pour obtenir un extrait de phycotoxine partiellement purifié ;
j) soumettre l'extrait partiellement purifié de l'étape précédente à une chromatographie en phase liquide à haute performance préparatoire en plusieurs étapes pour obtenir la phycotoxine biologiquement active pure.

2. Procédé selon la revendication 1, dans lequel l'extraction de l'étape c) est répétée 3 fois.

3. Procédé selon la revendication 1, dans lequel la solution de lavage des étapes f) et h) est de l'acide acétique 50 mM, dans l'étape f) la colonne est lavée avec 5 à 15 fois son volume et la solution d'élution est un mélange d'extraction alcoolique.

4. Procédé selon la revendication 1, dans lequel la solution d'élution de l'étape g) est un mélange d'élution alcoolique.

5. Procédé selon la revendication 1, dans lequel la solution de l'étape h) est un mélange de chloroforme:méthanol:eau 1:1:1 en vol/vol/vol.

6. Procédé selon la revendication 1, dans lequel les étapes chromatographiques de l'étape j) sont séquentiellement l'exclusion de taille, l'échange d'anions, l'échange de cations et l'exclusion de taille.

7. Procédé selon l'une quelconque des revendications 1 à 6, dans lequel les cyanobactéries sont aptes à produire des phycotoxines appartenant aux genres *Cylindrospermopsis sp*, *Microcystis sp, Anabaena sp, Gomphosphaeria sp*, *Oscillatoria sp*, *Aphanizomenon sp* et *Lyngbya wollei.*
